# NEW EUROPEAN PATENT SPECIFICATION

(11) **EP 1 237 608 B2**
(45) Date of publication and mention of the opposition decision: **19.05.2010**
(45) Mention of the grant of the patent: 29.11.2006
(21) Application number: 00989270.4
(22) Date of filing: 15.12.2000
(51) Int. Cl.: A61M 15/00

(54) **RECEPTACLES TO FACILITATE THE EXTRACTION OF POWDERS**
BEHÄLTER ZUR VEREINFACHUNG DER FÖRDERUNG VON PULVER
RECIPIENTS PERMETTANT L'EXTRACTION DE POUDRES

(30) Priority: 17.12.1999 US 172317 P
(43) Date of publication of application: 11.09.2002
(60) Divisional application: 06015709.6
(73) Proprietor: Novartis AG, 4056 Basel (CH)
(72) Inventor: PABOOJIAN, Steve, Menlo Park, CA 94025 (US); SCHULER, Carlos, Cupertino, CA 95014 (US); CLARK, Andrew, Woodside CA 94062 (US)
(74) Representative: Evens, Paul Jonathan
(86) International application number: PCT/US2000/034037
(87) International publication number: WO 2001/043529

(56) References cited:
- EP-A- 0 481 666
- EP-A- 1 106 196
- WO- -96/09085
- WO-A-98/34661
- WO-A-98/34663
- WO-A-98/34664
- CA-A- 2 316 345
- DE-A- 19 757 208
- US- - 3 967 761
- US- - 3 980 074
- US- - 5 622 166
- US-A- 5 239 991

## Description

This invention relates generally to techniques for extracting powdered medicaments from receptacles during the aerosolizing process.

One promising way to deliver various drugs to a patient is by pulmonary delivery where a drug dispersion or aerosol is inhaled by the patient to permit the active drug within the dispersion to reach the distal or alveolar regions of the lung. Pulmonary drug delivery has shown to be particularly promising because certain drugs have been found to readily absorb within the blood copulation. For example, pulmonary delivery may be a useful approach for proteins and polypeptides that are difficult to deliver by other routes of administration.

A variety of techniques have been employed to deliver drugs to the lungs including liquid nebulizers, metered dose inhalers, and the like. Of particular interest to the invention are dry powder dispersion devices that are able to aerosolize powdered medicaments for inhalation by the patient. Exemplary apparatus for aerosolizing powdered medicaments are described in U.S. Patent Nos. 5,458,135, 5,775,320, 5,740,794, 5,785,049, 6,089,228 and 6,257,233.

US 5,239,991 discloses a device for administering inhalation medicament, comprising a receptacle.

At least some of the apparatus described in the above references utilize a high pressure gas stream to draw the powder into an extraction tube where the powder is deagglomerated, entrained in the high pressure gas stream, and exits as an aerosol suitable for inhalation. In some cases, such apparatus may utilize a receptacle that has a penetrable lid. The extraction tube is inserted through the lid and a vent is also formed in the lid. The high pressure gas stream then dmws air through the receptacle and into the extraction tube. The air drawn through the receptacle extracts the powder where it joins with the high pressure gas stream to form the aerosol.

This invention is related to alternative ways to extract powder from receptacles that store the powder. The invention also relates to the design of such receptacles to facilitate removal of the powder.

### SUMMARY OF THE INVENTION

In accordance with the present invention, there is provided a system as defined in appendant claim 1, to which reference should now be made. Embodiments of this aspect of the present invention are defined in appendant dependent claims 2 - 4, to which reference should also now be made.

Such a configuration is advantageous in that it permits the flow of air to remain adjacent to an inner wall of the cavity until being drawn into an extraction tube. In this way, a generally laminar flow of air is provided across the walls of the cavity until reaching the extraction tube to provide a generally uniform shear stress across the walls. In this manner, the flow of air serves as a "scrubber" to remove powder from the walls where it may be drawn into the extraction tube.

The receptacle may have a which, in curved interior wall combination with the raised central region forms a generally semi-toroidal geometry in the cavity. With such a configuration, as the air is drawn through the cavity, it adheres to the walls and flows smoothly up the raised central region and into the extraction tube. Alternatively, the walls may be curved to form a "bow tie" configuration. Conveniently, a portion of the bottom end of the receptacle may be flat in geometry to facilitate convenient resting of the receptacle on a flat surface and accurate vertical positioning of me receptacle. The receptacle body may comprise a pair of opposing curved walls and a pair of opposing generally planar walls that at least partially form the cavity. Conveniently, the receptacle body may further include a tab that extends from the cavity to facilitate handling of the receptacle, e.g., such as when inserting the receptacle into an aerosolizing device.

Use of the raised central region is advantageous in that it channels or funnels the air toward the extraction tube to prevent powder from remaining in the center of the cavity.

The receptacle may have one or more curved interior walls. In this way, the air flows along the wall along substantially the entire length of the cavity to remove substantially all powder from the receptacle. By providing a wall with a continuous curvature, the momentum of the flowing air is changed which causes the air flow to stay adjacent the wall, causing powder adjacent the wall to be drawn into the extraction tube.

The air drawn by the gas stream may flow through a flow area that becomes progressively smaller. In this way, the air is accelerated as it flows through the receptacle and into the extraction tube to further assist in removing substantially all of the powder from the receptacle.

A hole may be pierced through the top end of the receptacle to permit the extraction tube to be inserted into the cavity through the hole in the top end. In one option, the gas stream may be introduced into the extraction tube at a location spaced apart from the bottom end of the extraction tube. Further, the gas stream may be introduced at an acute angle relative to a central axis of the extraction tube. In this way, the area of flow through the receptacle and the extraction tube may be controlled to accelerate the air through the receptacle. A hole may be formed in the bottom end of the receptacle body, and the gas stream may be flowed through the hole in the bottom end and then through the extraction tube, with outside air being drawn into the cavity through the vents to move the powder into the extraction tube. A flow insert may be provided to control spacing of the extraction tube relative to the receptacle. In this way, the area of flow through the receptacle and the extraction tube may be controlled to accelerate the air through the receptacle.

An amount of gas, preferably pressurized, is released to produce a high pressure gas stream that is utilized to extract the powder from the receptacle.

The system may further comprise a pressure source for producing a high pressure gas stream within at least a portion of the extraction tube. In this way, air may be drawn through the vents to move the powder from the cavity into the extraction tube where the powder is entrained in the gas stream to form an aerosol.

A flow insert may be provided to control spacing of the extraction tube relative to the receptacle. Further, the flow insert in combination with the vents and the extraction tube may be employed to accelerate the flow of air through the receptacle. For example, the vents may be configured to form a first flow area, and a gap between the extraction tube and the bottom end of the receptacle may define a second flow area A cross-section of the extraction tube may define a third flow area. In this way, the holder may be configured to move the receptacle relative to the bottom end of the extraction tube, or vice versa, such that the first flow area is greater than the second flow area, and the second flow area is greater than the third flow area.

Curved tabs or foils may be preformed in the top end of the receptacle body to create a vortex within the cavity as the air flows through the cavity. In this way, removal of substantially all the powder from the receptacle is facilitated. In another aspect, a portion of the bottom end of the receptacle may be flat in geometry to facilitate its placement onto the holder.

The receptacle may have a preformed central hole and vents about the periphery of the cavity. A cover may be removably attached to the top end of the receptacle. In this way, after the receptacle has been inserted into the aerosolizing apparatus, the cover may be pulled from the receptacle to permit the extraction tube to be inserted into the central hole and to expose the vents.

### BRIEF DESCRIPTION OF THE DRAWINGS

Fig.1 is a top view of a receptacle for holding a powder.
Fig. 2 is a cross sectional side view of the receptacle of Fig. 1, taken along lines 2-2.
Fig. 3 is a perspective view of the receptacle of Fig. 1 showing vents formed in a top end and an extraction tube that has been inserted into the top end.
Fig. 4 is a schematic side view of an exemplary method for extracting powder from the receptacle of Fig. 3.
Fig. 5 is a partial top view of another example of a receptacle having curved tabs or foils to produce a vortex in the receptacle when extracting powder.
Fig. 6 is a partial cross sectional side view of one of the tabs of the receptacle of Fig. 5.
Fig. 7 is a top view of an alternative receptacle.
Fig. 8A is a cross sectional side view of the receptacle of Fig. 7 taken along lines A-A.
Fig. 8B is a cross sectional side view of the receptacle of Fig. 7 taken along lines B-B.
Fig. 9 is a perspective view of a receptacle into which an extraction tube is inserted and illustrates the various flow areas through which the air flows when extracting the powder from the receptacle.
Fig. 10 is a schematic side view of an aerosolizing device that may be employed to aerosolize a powder.
Fig. 11 is a schematic side view of a receptacle and an extraction tube and illustrates an alternative technique for extracting powder.
Fig. 12 is a schematic side view of an alternative aerosolizing device.
Fig.13 is a top view of still another alternative receptacle having a removable cover.
Fig. 14 is a cross sectional side view of the receptacle of Fig.13.
Fig. 15 is a schematic side view of a breath actuated aerosolizing device.

### DESCRIPTION OF THE SPECIFIC EMBODIMENTS

There are provided exemplary techniques and equipment for extracting powder that is held within a receptacle, typically within a sealed cavity. In one aspect, the powder extracted is entrained in a high pressure gas stream to aerosolize the powder so that it may be suitable for inhalation by a patient. A receptacle within which the powder is sealed may be used. Merely by way of example, one type of receptacle that may be utilized with the invention are widely available "blister packs". Examples of other types of receptacles are described in U.S. Patent No. 5,740,794.

The powders may be extracted by creating an opening or access way into the receptacle and then flowing air or other gases through the receptacle to move the powder out of the access way. Vents are also created in the receptacle to facilitate the flow of air through the receptacle. One exemplary way to draw air through the receptacle is by use of an extraction tube that is inserted into the cavity. A high pressure gas stream is flowed through at least a portion of the extraction tube to cause air in the receptacle to be drawn into the bottom end of the extraction tube where the powder is entrained in the high pressure gas stream to form an aerosol. Examples of techniques that employ the use of such an extraction tube are described in U.S. Patent No. 5,740,794. Further, a variety of techniques may be employed to create the high pressure gas stream to cause the air to be drawn through the receptacle. For example various techniques for producing the high pressure gas stream are described in U.S. Patent Nos 5,740,794, 6,089,228 and 6,257,233. Gases that may be used to produce the gas stream include air, CO₂, HFCs, CFCs, and the like.

To draw air through the receptacle and into the bottom end of the extraction tube, the high pressure gas stream may be introduced into the extraction tube at a location that is spaced apart from the bottom end. For example, the high pressure gas stream may be introduced into the extraction tube at an acute angle as described generally in U.S. Patent No. 5,740,794. Alternatively, a hole may be formed in the bottom end of the receptacle along with one or more vents, and the extraction tube inserted into the top end of the receptacle so that it is generally aligned with the hole. The high pressure gas stream may then be flowed through the hole and into the extraction tube to cause air to be drawn through the vents, through the receptacle and into the bottom end of the extraction tube.

A variety of schemes may be employed, alone or in combination, to facilitate the extraction of the powder using air flowing through the receptacle. For example, one technique employs the use of air or other gases to uniformly "scrub" the sides of the cavity. More specifically, the air may be flowed adjacent an interior wall or walls until the air exits the receptacle through the extraction tube. In this way, a shear stress is provided along substantially the entire length of the interior wall to assist in removing any powder that adheres to the wall so that it may be moved into the extraction tube. The walls may be constructed with a variety of geometries to facilitate a laminar flow across the walls so that the flow will not become separated from the walls as it flows through the receptacle. In this way, a uniform, "scrubbing" of the walls is provided. Merely by way of example, one convenient way to form the walls is to provide them with a degree of curvature so that they continuously curve up to the extraction tube. Such a continuous curved surface allows for a laminar flow along substantially the entire length of the walls and up to the extraction tube. The curvature of the walls also tends to induce instabilities that are manifested as a plurality of counter-rotating vortices, sometimes referred to as Taylor-Goertler vortices, having axes of rotation locally parallel to the curved walls. These vortices serve to scrub the walls of powder. The receptacle includes a raised region at a center of the receptacle so that the walls slope up to the extraction tube. In this way, no dead space is provided in the middle of the receptacle and the flow remains adjacent to the walls until exiting through the extraction tube.

Another technique to facilitate removal of the powder is to accelerate the flow of air through the receptacle. One convenient way to accelerate the air flow is to progressively decrease the area through which the air passes as it flows through the receptacle and out of the extraction tube. By progressively reducing the flow area, the air is accelerated as it flows through the receptacle and into the extraction tube.

Still another technique for facilitating the removal of the powder is to create a vortex in the cavity to permit the air to sweep the sides of the receptacle as it spins through the cavity and up into the extraction tube. Conveniently, curved edges, tabs or foils may be formed in the top end of the receptacle to initiate the vortex when air is drawn into the receptacle.

The receptacles may be constructed to have preformed holes and/or vents. In this way, the top surface of the receptacle does not need to be pierced when inserting the extraction tube or forming the vents. Conveniently, a removable cover may be placed on top of the receptacle. After insertion into an aerosolizing apparatus, the cover may be pulled from the receptacle to expose the holes and/or vents. The extraction tube is then inserted into the receptacle and the powder extracted as described herein.

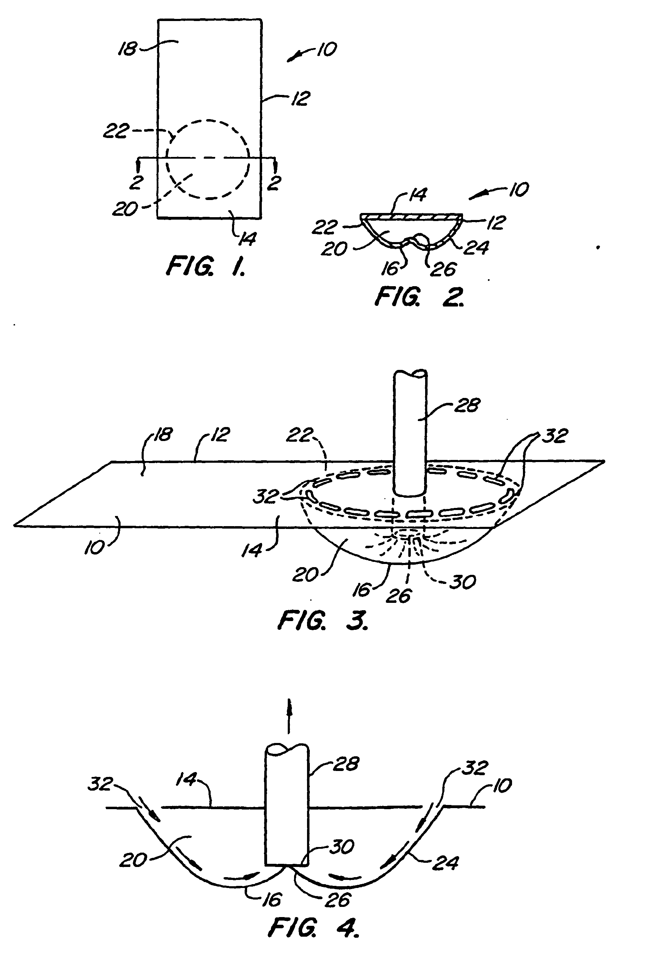

Referring now to Figs. 1 and 2, a receptacle 10 will be described. Receptacle 10 comprises a receptacle body 12 having a top end 14 and a bottom end 16 (see Fig. 2). Conveniently, a tab 18 may be provided to facilitate handling of receptacle 10. Receptacle body 12 defines a cavity 20 into which a powder is sealed. Conveniently, receptacle body 12 may be constructed from essentially any type of material that is compatible with the powder held within cavity 20. Examples of materials that may be used include metals, such as aluminum, composites, plastics, and the like. One convenient way to construct receptacle 10 is to provide a thin strip of metal or composite and then pressing cavity 20 using a die. Another thin strip of metal may then be attached to the strip having the cavity to enclose and seal the cavity. Conveniently, ultrasonic welding or heat sealings may be employed to adhere the two metal shrns together. However, it will be appreciated that other techniques and materials may be employed to construct receptacle 10. Further, a number of receptacles may be formed as a single string for multi-dose aerosolizers.

Cavity 20 has a generally circular outer periphery 22 and is formed of a continuously curved wall 24 that forms a raised central region 26 at or near a center of the receptacle. In this way, a generally semi-toroidal interior is formed.

Referring now to Fig. 3, an extraction tube 28 is shown inserted into cavity 20. Extraction tube 28 has a bottom end 30 that is generally aligned with raised central region 26 when inserted into receptacle 10. Conveniently, bottom end 30 of extraction tube 28 may include a sharpened edge to facilitate its entry into cavity 20. Alternatively, a preformed hole may be formed in top end 14 to permit entry of extraction tube 28 into cavity 20. Bottom end 30 is positioned so that it is spaced apart from bottom end 16 of cavity 20. In this way, a gap is provided between bottom end 30 and bottom end 16 to permit air to flow between the gap. Also shown in Fig. 3 are a plurality of vents 32 that are formed about periphery 22. In one aspect, vents 32 may be formed such that they are spaced close to each other in an attempt to form an annulus about periphery 22. In this way, air may be introduced into cavity 20 about essentially the entire periphery 22.

Referring now to Fig. 4, one technique for extracting powder from receptacle 10 using extraction tube 28 will be described. A high pressure gas stream (not shown) is flowed past a portion of extraction tube 28 at a location spaced above bottom end 30 as described generally in U.S. Patent No.5,740,794. This causes air to be drawn into receptacle 10 through vents 32 as illustrated by the snows. The air is flowed through cavity 20 until entering bottom end 30 where it proceeds through extraction tube 28. Eventually, the air containing the powder is joined with the high pressure gas stream that deagglomerates the powder and entrains the powder in the gas stream to form an aerosol.

As shown in Fig. 4, wall 24 has a continuous curvature so that the air flowing through cavity 20 remains generally adjacent wall 24 with a laminar flow. In this way, a shear stress is created along substantially the entire length of wall 24 to remove any adhered powder along wall 24. Further, central region 26 directs the air up to bottom end 30 so that essentially no dead volume exists within cavity 20. In this manner, substantially all the powder is drawn into extraction tube 30. It will be appreciated that the size of cavity 20 as well as the degree of curvature of wall 24 may be varied depending on a variety of features, including, for example, the volume and rate of air flow through cavity 20, the amount and type of powder held within cavity 20, and the like.

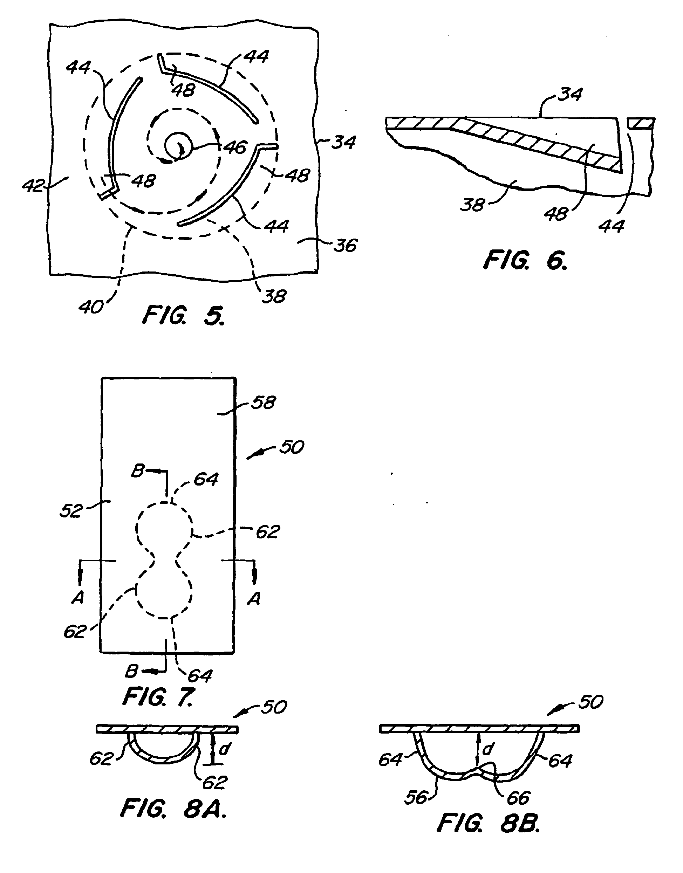

Referring now to Figs. 5 and 6, another example of a receptacle 34 will be described. Receptacle 34 comprises a receptacle body 36 that forms a cavity 38 (shown in phantom line). Cavity 38 has a generally circular outer periphery 40 and includes a raised central region as already described. Formed in a top end 42 of receptacle body 36 are a plurality of vents 44. A central hole 46 is also formed in top end 42 and is configured to receive an extraction tube. In this way, air may be drawn through vents 44, through cavity 38, and into the extraction tube where it is extracted from the receptacle in a manner similar to that previously described.

One feature of receptacle 34 is that it includes curved foils 48 at each of the vents 44. As best shown in Fig. 6, foils 48 extend into cavity 38 so as to produce a vortex within cavity 38 when air is drawn in through vents 44. This is illustrated generally by the arrows in Fig. 5. By creating a vortex within cavity 38, the air flow is swirled around the interior walls that define cavity 38 to assist in removing powder that is adhered to the walls. Advantageously, the vortex results in an acceleration of the air to further assist in removing powder from the receptacle. Further, large powder agglomerates within cavity 38 may be caught in the vortex and thrown radially outward because of centripetal acceleration coincident with their larger mass relative to smaller powder particles. In this way, the larger agglomerates are statistically more likely to strike the side of the tube to induce deagglommeration.

Figs. 7,8A and 8B illustrate another receptacle 50. Receptacle 50 comprises a receptacle body 52 having a top end 54, a bottom end 56 and a tab 58. Receptacle body 52 defines a cavity 60 into which a powder is held. Cavity 60 is defined by two side walls 62 and two end walls 64 to form a "bow tie" configuration. A raised central region 66 extends up into cavity 60 in a manner similar to raised central region 26 of receptacle 10.

To extract powder from receptacle 50, an extraction tube (not shown) is inserted through top end 54 and aligned above raised central region 66 in a manner similar to that previously described in connection with receptacle 10. Vents are then formed in top end 54 adjacent curved walls 64. In this manner, air will be drawn through the vents and along curved wall 64 where the air will be funneled by raised central region 66 into the bottom end of the extraction tube. By providing curved walls 64, the air flow will tend to flow along the walls to assist in removing powder that adheres to the walls in a manner similar to that previously described in connection with receptacle 10.

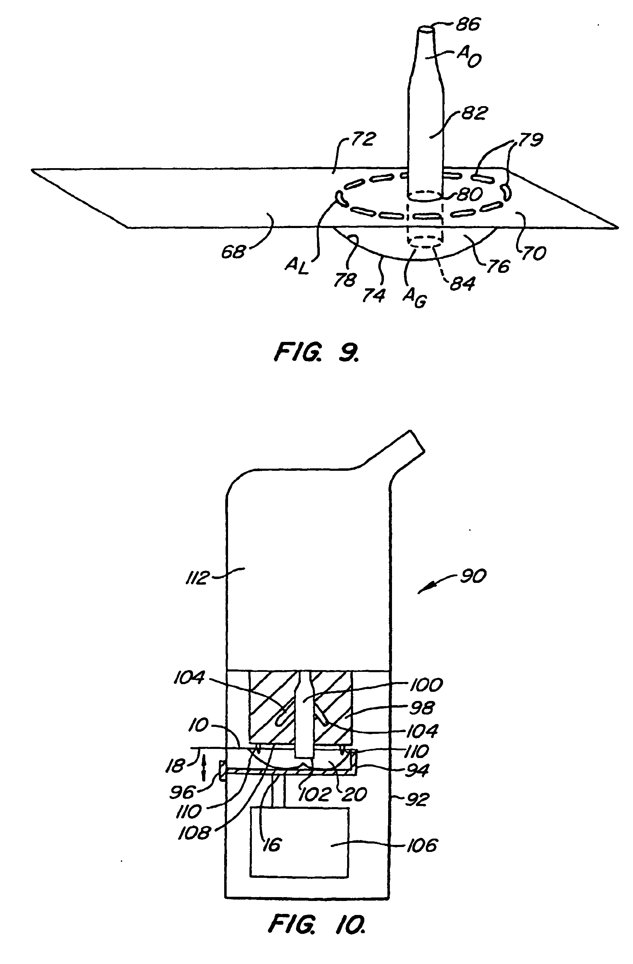

Another technique that may be employed to facilitate extraction of the powder is by accelerating the flow of air through the cavity. Fig. 9 illustrates one technique for accelerating the flow of air through the cavity. Shown in Fig. 9 is a receptacle 68 comprising a receptacle body 70 having a top end 72 and a bottom end 74. Receptacle body 68 forms a cavity 76 that is defined by an interior wall 78. Cavity 76 has a raised central region as previously described. A plurality of vents 79 are formed in top end 72 about a periphery of cavity 76. A center hole 80 is also provided in top end 72 to permit an extraction tube 82 to be inserted into cavity 76 as shown. Extraction tube 82 has a bottom end 84 and a top end 86. Optionally, extraction tube 82 may have a reduced cross sectional area at top end 86 to facilitate aerosolization of the powder as described generally in U.S. Patent No. 5,740,794. Although not shown, it will be appreciated that a high pressure gas stream may be flowed past a portion of extraction tube 82 at a location spaced apart from bottom, end 84 in a manner similar to that previously described. Alternatively, the high pressure gas stream may be flowed through a hole in bottom end 74 and then into extraction tube 82, as described hereinafter. In either case, the use of such a high pressure gas stream causes air to be drawn into cavity 76 through vents 79 where the powder is moved into extraction tube 82 through its bottom end 84 where it is entrained in the high pressure gas stream and aerosolized.

Each of vents 79 forms a flow area Aᵢ. When summed together, areas Aᵢ form a total input flow area A_{I}. As the air passes through cavity 76, it flows through a gap created between bottom end 84 of extraction tube 82 and bottom end 74 of cavity 76. This flow area may be calculated by multiplying the distance of the gap by the circumference of extraction tube 82 at bottom end 84. This area is referred to as the gap area A_{G}. As the air flows through extraction tube 82, the flow area is restricted near top end 86 as shown. This area is the cross sectional area A_{O} of the extraction tube. To accelerate the flow of air through cavity 76, areas A_{I}, A_{G} and A_{O} may be configured such that A_{I} > A_{G} > A_{O}. In this way, the flow area is progressively decreased as it passes through the system. As such, the flow of air is accelerated as it passes through cavity 76. Although a variety of area ratios may be employed, one particular ratio is where A_{I} = 2, A_{G} = 1.5, and A_{O} = 1. However, it will be appreciated that other ratios may be employed.

Referring now to Fig. 10, an aerosolization apparatus 90 will be described. Apparatus 90 comprises a base 92 that forms a housing for various components of apparatus 90. Enclosed in base 92 is a holder 94 for holding a receptacle. For convenience of illustration, receptacle 10 of Fig. 1 is shown held within base 92. However, it will be appreciated that other types of receptacles may be utilized with apparatus 90. A button 96 is provided on holder 94 to permit holder 94 to move up and down within base 92 as indicated by the arrows. As shown, holder 94 has a generally flat surface. As previously described, receptacle body 12 may be included with a flat portion on bottom end 16 to facilitate its placement onto holder 94. However, it will be appreciated that holder 94 may be constructed to have different geometries to facilitate holding of receptacle 10, as well as to facilitate introduction and removal of receptacle 10.

Positioned above receptacle 10 is an aerosolizing mechanism 98 that includes an extraction tube 100 that is insertable into cavity 20 of receptacle 10. Optionally, a bottom end 102 of extraction tube 10D may include a sharpened edge or other piercing structure to form a hole in the top end of receptacle 10 to facilitate its introduction into cavity 20. Joining extraction tube 100 at an acute angle relative to a central axis of extraction tube 100 (and relative to bottom end 102) are a pair of channels 104. A pressure source 106 is employed to produce a high pressure gas stream within channels 104. The high pressure gas stream is introduced into extraction tube 100 to cause air to be drawn into bottom end 102 from cavity 20 as described generally in U.S. Patent No. 5,740,794. Pressure source 106 may be any one of a variety of pressure sources, including manually activated pistons, compressed gases, fluorocarbons, and the like as described generally in the patents previously referred to. Hence, it will be appreciated that pressure source 106 is merely being shown schematically for convenience of illustration. Although not shown, an actuating mechanism may be employed to release the pressurized gas from pressure source 106 when a patient is ready to produce the aerosolized medicament.

Aerosolization mechanism 98 includes a bottom end 108 that serves as a stop or a flow insert to control the gap between bottom end 102 of extraction tube 100 relative to bottom end 16 of receptacle 10. In this way, when button 96 is moved upward, the top end of receptacle 10 will engage bottom end 108 to fix the distance of extraction tube 100 relative to the bottom end of receptacle 10. Use of such a flow insert is advantageous in that the gap area A_{G} (see Fig. 9) may be precisely controlled to facilitate the acceleration of air through cavity 20 in a manner similar to that previously described in connection with Fig. 9.

Extending from bottom end 108 are a plurality of piercing elements 110 that are configured to produce vents in receptacle 10 about the periphery of cavity 20. In this way, air may be drawn through the vents when the high pressure gases stream is released from pressure source 106.

Coupled to base 92 is a capture chamber 112. Capture chamber 112 is configured to capture the aerosolized medicament exiting extraction tube 100. Capture chamber 112 includes a mouthpiece 114 through which the patient may inhale the captured medicament.

Hence, apparatus 90 may be employed to aerosolize a medicament by inserting receptacle 10 into base 92. Holder 94 is then raised to insert extraction tube 102 into cavity 20 and to cause piercing elements 110 to form vents in receptacle 10. Pressure source 106 is actuated to release an amount of pressurized gas which causes air to be drawn into and through the vents and along the walls of cavity 20 until entering into extraction tube 100. As the powder is moved into extraction tube 100, it encounters the high pressure gas stream which deagglomerates the powder and ejects the powder into capture chamber 112 in an aerosolized form. Although holder 94 is shown to move vertically upward, it will be appreciated that extraction tube 100 and/or piercing elements 110 may be configured to be moved downward to be inserted into cavity 20. Further, alternative aerosolizing mechanisms 98 may be employed as described herein.

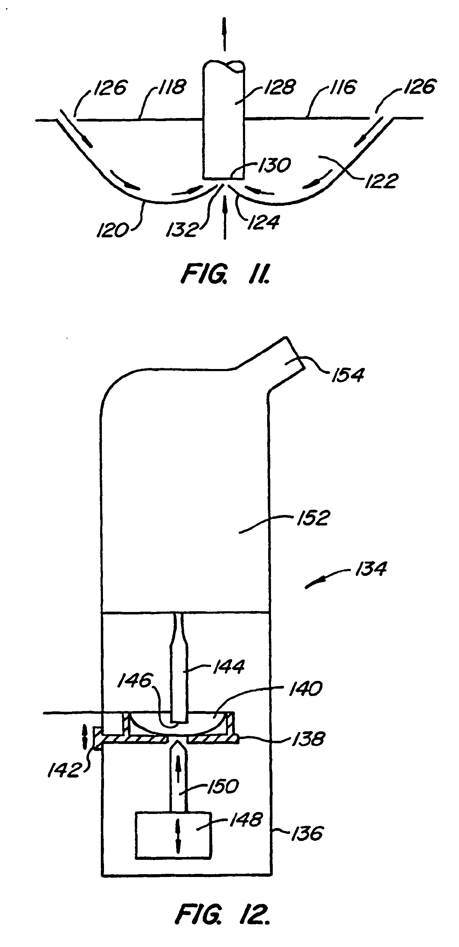

Referring now to Fig. 11, an alternative technique for drawing air through a receptacle to move powder within the receptacle into an extraction tube will be described. Shown schematically in Fig. 11 is a receptacle 116 having a top end 118 and a bottom end 120. Receptacle 116 includes a cavity 122 having a raised central region 124. A plurality of vents 126 are formed in top end 118 to permit air to be drawn into cavity 122. An extraction tube 128 is inserted into cavity 122, with a bottom end 130 being spaced apart from raised central region 124 as shown. A bottom hole 132 is formed in bottom end 120 of receptacle 116. In this way, a high pressure gas stream may be flowed through bottom hole 132 and then through bottom end 130 of extraction tube 128, as shown by the arrows. In so doing, air is drawn through vents 126 and through cavity 122 where it enters bottom end 130 of extraction tube 128 as shown by the arrows. As the air flows through cavity 122, it moves the powder into extraction tube 128 in a manner similar to that previously described in connection with other embodiments.

Shown schematically in Fig. 12 is an aerosolizing apparatus 134 that may be employed to aerosolize a powdered medicament using the techniques just described in connection with Fig. 11. Apparatus 134 comprises a base 136 having a holder 138 for holding a receptacle 140. Holder 138 includes a knob 142 to permit receptacle 140 to be moved up and down as shown by the arrows. Also included within base 136 is an extraction tube 144 having a bottom end 146. By moving knob 142, extraction tube 144 may be inserted into receptacle 140 as shown. Alternatively, extraction tube 144 may be constructed to be movable so mat it may be moved into receptacle 140.

Positioned below holder 13 8 is a pressure source 148 and an introduction tube 150. Pressure source 148 and/or introduction tube 150 may be moved vertically upward as illustrated by the arrows to pierce receptacle 140 and insert introduction tube 150 into or adjacent to receptacle 140. An amount of pressurized gas may then be released from pressure source 150 where it flows through the hole in the bottom end of receptacle 140 and into bottom end 146 of extraction tube 144. As one alternative, holder 142 may be lowered while pressure source 148 is kept stationary to form the hole in the bottom end of receptacle 140. Although not shown, it will be appreciated that a piercing mechanism may be employed to form one or more vents in receptacle 140 (or receptacle 140 may include pre-formed vents). In this way, outside air may flow into the receptacle through the vents to assist in moving powder into tube 144.

Positioned on base 136 is a capture chamber 152 having a mouthpiece 154. With such a configuration, receptacle 140 may be placed into holder 138 and extraction tube 144 inserted into receptacle 140. A hole may then be formed in the bottom end of receptacle 140 and a pressurized gas from pressure source 148 released to cause a high pressure gas stream to flow through extraction tube 144, In so doing, air is drawn into and through the receptacle and into extraction tube 144 where the powder is aerosolized and ejected into capture chamber 152.

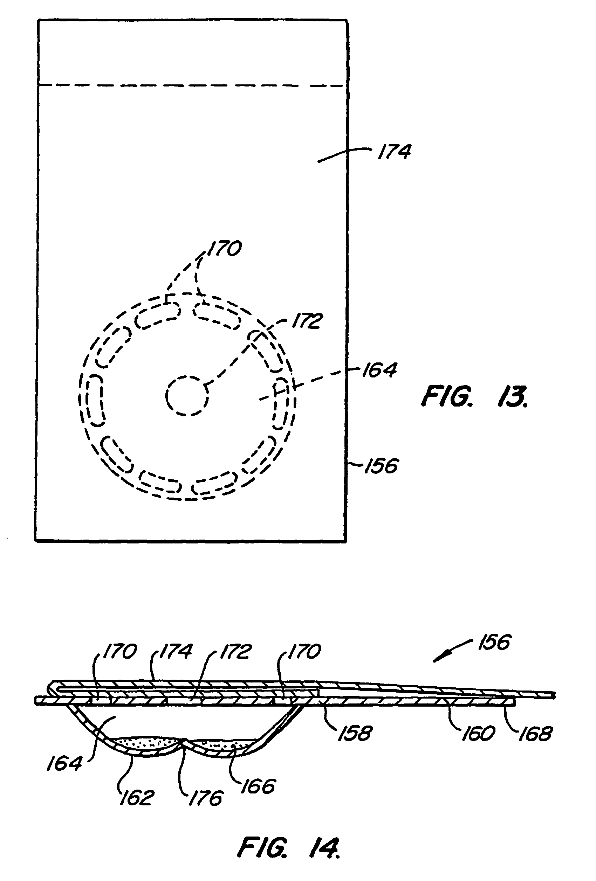

Shown in Figs. 13 and 14 is an alternative receptacle 156. Receptacle 156 comprises a receptacle body 158 having a top end 160 and a bottom end 162. Receptacle body 158 forms a cavity 164 that holds a powder 166. Extending from cavity 164 is a tab 168 to facilitate handling of the receptacle. Formed about a periphery of cavity 164 are a plurality of vents 170 that extend through top end 160. Extending through top end 160 at a center of cavity 164 is a hole 172 that is adapted to receive an extraction tube (not shown) in a manner similar to that described previously.

Secured to top end 160 at a location above cavity 164 is a cover 174. Cover 174 is secured to top end 160 in a manner such that vents 170 and hole 172 is covered to seal powder 166 within cavity 164. As shown, cavity 164 includes a raised central region 176. Cover 174 is folded over itself and extends back over tab 168. In this way, cover 174 will generally extend outside of an aerosolization apparatus. As such, when the user is ready to aerosolize the medicament, receptacle 156 is inserted into the aerosolization apparatus and cover 174 is pulled from top end 160. In this way, vents 170 and hole 172 are exposed An extraction tube may then be inserted through hole 172 in a manner similar to that previously described. By preforming vents 170 and 172, top end 160 will not need to be pierced while within the aerosolization apparatus.

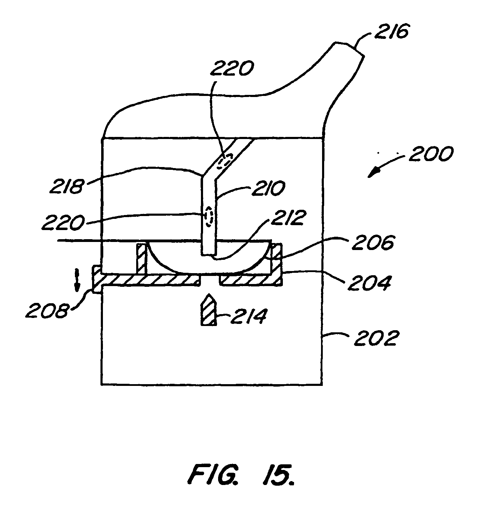

Shown schematically in Fig. 15 is an aerosolizing apparatus 200 that may be employed to aerosolize a powdered medicament. Apparatus 200 comprises a base 202 having a holder 204 for holding a receptacle 206, which is representative of any of the receptacles described herein. Holder 204 includes a knob 208 to permit receptacle 206 to be moved up and down as shown by the arrows. Also included within base 202 is an extraction tube 210 having a bottom end 212. By moving knob 208, extraction tube 210 may be inserted into receptacle 206 as shown. Alternatively, extraction tube 210 may be constructed to be movable so that it maybe moved into receptacle 206. Conveniently, a piercing mechanism 214 may be positioned below holder 204 to permit a hole to be pierced in the bottom of receptacle 206 when knob 208 is lowered. Positioned on top of base 202 is a mouthpiece 216 over which a patient's mouth may be placed when ready to receive a dose of medication.

Optionally, extraction tube 210 may have one or more bends 218 to facilitate powder deagglomeration as the power passes through extraction tube 210. As another option, one or more obstacles 220 may be placed into extraction tube 210 to facilitate powder deagglomeration. Further, it will be appreciated that bends and obstacles may be provided in the extraction tubes of the other aerosolization devices described herein.

The invention has now been described in detail for purposed of clarity of understanding. However, it will be appreciated that certain changes and modifications may be practiced within the scope of the appended claims.

## Claims

1. A system for aerosolizing a powdered medicament, the system comprising:
at least one receptacle (10), (34), (50), (116), (156) for holding fine powders comprising a receptacle body (12) (36) (52) (158) that defines an enclosed cavity (20) (38) (60) (122) (164) in which the powdered medicament is held, the receptacle body having a top end (14) (42) (54) (118) (160) and a bottom end (16) (56) (120) (162), with the bottom end (16) (56) (120) (162) of the receptacle body including a raised central region (26) (66) (124) (176) that extends upwardly into the cavity (20) (38) (60) (122) (164); and
an aerosolizing apparatus (90) having a holder (94) for holding the receptacle (10), (34), (50), (116), (156), an extraction tube (100) that is insertable into the cavity (20), (38), (60), (122), (164), and a vent forming device (110) to form multiple vents in the top end of the receptacle about a periphery of the cavity.

2. A system as in claim 1, further comprising a pressure source (106) for producing a high pressure gas stream within at least a portion of the extraction tube to draw air through the vents to move the powder from the cavity and into the extraction tube where the powder is entrained in the high pressure gas stream to form an aerosol.

3. A system as in claim 1, further comprising a flow insert (108) to control spacing of the extraction tube relative to the receptacle.

4. A system as in claim 1, wherein a portion of the bottom end of the receptacle is flat in geometry.

## Patentansprüche

1. System zum Bringen eines pulverförmigen Medikaments in Aerosolform, wobei das System aufweist:
wenigstens einen Behälter (10), (34), (50), (116), (156) zum Aufnehmen feiner Pulver, umfassend einen Behälterkörper (12)(36)(52)(158), der eine umschlossene Höhlung (20)(38)(60)(122)(164) begrenzt, in der das pulverförmige Medikament gehalten wird, wobei der Behälterkörper ein oberes Ende (14)(42)(54)(118)(160) und ein unteres Ende (16)(56)(120)(162) hat, wobei das untere Ende (16)(56)(120)(162) des Behälterkörpers einen erhöhten mittleren Bereich (26)(66)(124)(176) aufweist, der sich nach oben in die Höhlung (20)(38)(60)(122)(164) erstreckt; und
eine Aerolisiervorrichtung (90) mit einem Halter (94) zum Halten des Behälters (10), (34), (50), (116), (156), einem in die Höhlung (20)(38)(60)(122)(164) einführbaren Absaugrohr (110) sowie einer öffnungsbildenden Einrichtung (110) zum Bilden mehrerer Öffnungen in dem oberen Ende des Behälters um einen Rand der Höhlung herum.

2. System nach Anspruch 1, ferner aufweisend eine Druckquelle (106) zum Erzeugen eines Hochdruckgasstroms in zumindest einem Teil des Absaugrohrs, um Luft durch die Öffnungen zu saugen zum Bewegen des Pulvers aus der Höhlung und in das Absaugrohr, wo das Pulver in dem Hochdruckgasstrom zur Bildung eines Aerosols mitgerissen wird.

3. System nach Anspruch 1, ferner aufweisend einen Strömungseinsatz (108) zum Steuern des Abstandes des Absaugrohrs relativ zum Behälter.

4. System nach Anspruch 1, bei dem ein Teil des unteren Endes des Behälters eine ebene Geometrie hat.

## Revendications

1. Système de production d'aérosol d'un médicament pulvérulent, le système comprenant:
au moins un récipient (10), (34), (50), (116), (156) pour contenir des poudres fines comprenant un corps de récipient (12) (36) (52) (158) qui définit une cavité fermée (20) (38) (60) (122) (164) dans laquelle le médicament pulvérulent est contenu, le corps de récipient ayant une extrémité supérieure (14) (42) (54) (118) (160) et une extrémité inférieure (16) (56) (120) (162), alors que l'extrémité inférieure (16) (56) (120) (162) du corps de récipient comporte une région centrale surélevée (26) (66) (124) (176) qui s'étend vers le haut jusque dans la cavité (20) (38) (60) (122) (164) ; et
un appareil de production d'aérosol (90) ayant un support (94) pour supporter le récipient (10), (34), (50), (116), (156), un tube d'extraction (100) qui peut être inséré dans la cavité (20), (38), (60), (122), (164), et un dispositif de formation d'aérations (110) pour former de multiples aérations dans l'extrémité supérieure du récipient autour d'un contour de la cavité.

2. Système tel que défini dans la revendication 1, comprenant en outre une source de pression (106) pour produire un courant gazeux à haute pression au sein d'au moins une partie du tube d'extraction pour aspirer l'air à travers les aérations afin de déplacer la poudre depuis la cavité et jusque dans le tube d'extraction où la poudre est entraînée dans le courant gazeux à haute pression pour former un aérosol.

3. Système tel que défini dans la revendication 1, comprenant en outre un insert d'écoulement (108) pour contrôler l'espacement du tube d'extraction relativement au récipient.

4. Système tel que défini dans la revendication 1, dans lequel une partie de l'extrémité inférieure du récipient est plate en géométrie.
